# EUROPÄISCHE PATENTSCHRIFT

(11) **EP 0 521 239 B1**
(45) Veröffentlichungstag und Bekanntmachung des Hinweises auf die Patenterteilung: **30.11.1994**
(21) Anmeldenummer: 92102895.7
(22) Anmeldetag: 21.02.1992
(51) Int. Cl.: B01D 53/04, C07C 17/38

(54) **Verfahren zur Rückgewinnung von in einem Adsorber adsorbierten Lösungsmitteln**
Process for the recovery of solvents adsorbed in an adsorber
Procédé de récupération de solvants adsorbés dans un adsorbeur

(30) Priorität: 29.06.1991 DE 4121697
(43) Veröffentlichungstag der Anmeldung: 07.01.1993
(73) Patentinhaber: PERO KG, 86343 Königsbrunn (DE)
(72) Erfinder: Erbel, Horst, W-8900 Augsburg 21 (DE)
(74) Vertreter: Charrier, Rolf, Dipl.-Ing.

(56) Entgegenhaltungen:
- EP-A- 0 381 942
- DATABASE WPI Section Ch, Week 7644, Derwent Publications Ltd., London, GB;Class J01, AN 76-81904X

## Beschreibung

Die Erfindung betrifft ein Verfahren zur Rückgewinnung von in einem Adsorber adsorbierten umweltbelastenden Stoffen, wie beispielsweise Lösungsmitteln.

Die DE-A-30 48 649 beschreibt ein Verfahren zur Rückgewinnung von in Aktivkohle adsorbierten halogenierten Kohlenwasserstoffen. Bei diesem Verfahren wird die Aktivkohle von Heißluft durchströmt, wobei die adsorbierten Kohlenwasserstoffe desorbiert werden und in die durchströmende Luft übergehen. Die abströmende Luft wird sodann gekühlt, was zu einer Kondensation der darin gelösten Kohlenwasserstoffe führt. Durch erneute Erhitzung und Wiederzuführung der Luft in die Aktivkohle kann diese Prozedur mehrfach wiederholt werden. Es ist jedoch immer streng darauf achten, daß die Temperatur der Luft unterhalb der Zersetzungstemperatur der halogenierten Kohlenwasserstoffe liegt. Die obere Grenze für die Temperatur der durchströmenden Heißluft beträgt beispielsweise beim Trichlorethylen 120°C und beim Perchlorethylen 150°C. Diese Werte gelten für normale, atmosphärische Umgebung. Ein Luftdruck von ca. 1000 mbar sei dabei als normaler Luftdruck bezeichnet.

Bei diesem Verfahren ist nachteilig, daß trotz mehrfacher Wiederholung der Prozedur keine vollständige Desorption der Kohlenwasserstoffe vom Aktivkohlefilter stattfindet. Weiterhin weist die Luft nach Durchführung des Verfahrens eine Restmenge des Kohlenwasserstoffs auf.

Weiterbildungen dieses Verfahrens sind in den Patent Abstracts of Japan, Unexamined Applications, Section C, Band 2, Hr. 59, 27. April 1978, Seite 458 C 78, Nr. 53-18504 und in der EP-A-0 381 942 veröffentlicht. In diesen Schriften wird die Desorption der adsorbierten Kohlenwasserstoffe durch Anlegen eines Unterdrucks an den Adsorberraum unterstützt. In der EP-A-0 381 942 wird dabei zunächst der Adsorber auf eine Temperatur erhitzt, die unter der Zersetzungstemperatur des adsorbierten Stoffs liegt, daraufhin wird der Adsorberraum gegenüber der Umgebung abgeschlossen und ein hoher Unterdruck angelegt. Schließlich wird der desorbierte Stoff vom Adsorberraum abgezogen und die Temperatur- und Druckverhältnisse so eingestellt, daß er kondensiert.

Dieses unterdruckunterstützte Verfahren ist zwar dem erstgenannten überlegen. Es ermöglicht jedoch auch keine vollständige Desorption, da wiederum die maximale Arbeitstemperatur durch die Zersetzungstemperatur des adsorbierten Stoffs begrenzt ist. Der Einsatz höherer Temperaturen, bei welchen eine wesentlich bessere Desorption stattfinden würde, ist daher nicht möglich.

Es besteht daher die Aufgabe, das Verfahren so auszubilden, daß eine nahezu vollständige Desorption des adsorbierten Stoffs ermöglicht wird.

Gelöst wird diese Aufgabe mit den Merkmalen des Anspruchs 1. Vorteilhafte Ausgestaltungen des Verfahren sind den Unteransprüchen entnehmbar.

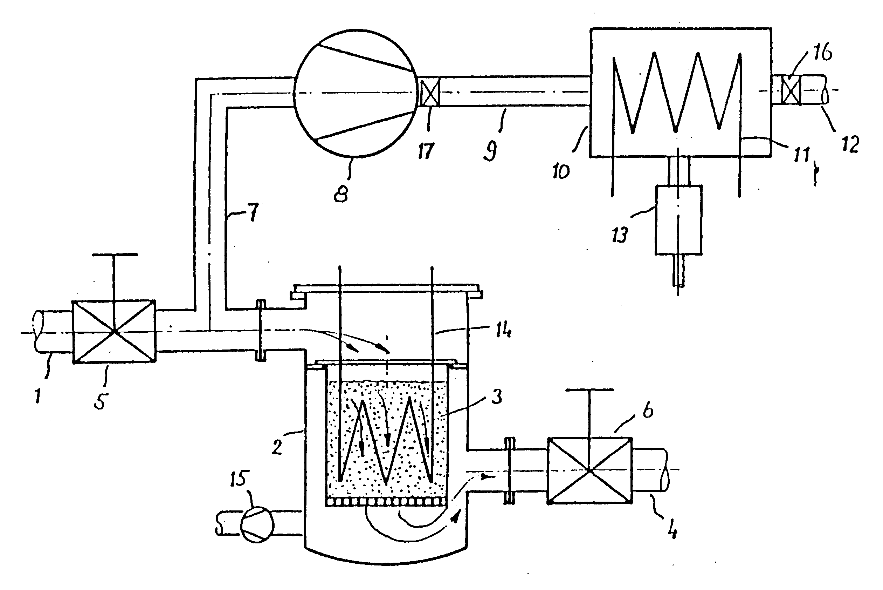

Das Verfahren soll im folgenden anhand der Zeichnung, welche eine Ausführungsform der dazu verwendeten Vorrichtung schematisch darstellt, erläutert werden.

Die mit einem organischen Lösungsmittel, wie beispielsweise Trichlorethylen, Perchlorethylen oder Trichlorethan, versetzte Luft gelangt über die Rohrleitung 1 zum Behälter 2, in dem sich der Adsorber 3, beispielsweise ein Aktivkohlefilter, befindet. Nach Durchströmung des Adsorbers 3 verläßt die gereinigte Luft diesen über die Rohrleitung 4. Die Rohrleitungen 1 und 4 weisen jeweils einen Absperrschieber 5, 6 auf. Die Rohrleitung 1 weist einen Abzweig zu einer Rohrleitung 7 auf, welche zu einer Vakuumpumpe 8 führt. An der anderen Seite dieser Vakuumpumpe ist ein, aus einem Kühlbehälter 10 und einem Kühler 11, bestehendes Kühlsystem angeordnet, welches auch einen Abscheider 13 aufweist. Im Adsorber 3 ist eine Heizvorrichtung 14 angeordnet.

Wenn der Adsorber 3 mit Lösungsmittel angereichert ist, weist er keine Filterfunktion mehr auf und muß desorbiert werden. Dazu wird er auf eine Temperatur erhitzt, die zunächst unter der Zersetzungstemperatur des adsorbierten Stoffes liegt. Sodann wird über die Vakuumpumpe 8 der Adsorberraum evakuiert, was in bekannter Weise die Desorption unterstützt. Der typische Restdruck im Adsorberraum beträgt 10 bis 100 mbar.

Erfindungsgemäß kann nun unter weiterer Senkung des Drucks bis unter 1 mbar die Temperatur des Adsorbers 3 auf Werte oberhalb der Zersetzungstemperatur des Lösungsmittels erhöht werden, ohne daß sich das Lösungsmittel dabei zersetzt. Dies ist ein ausgesprochen überraschender Effekt. In der Fachwelt galt bislang die Zersetzungstemperatur des Lösungsmittels als absolute Obergrenze der Erhitzung.

Auch die thermische Zerstörung anderer organischer Substanzen im Adsorberraum 2 wird durch das erfindungsgemäße Verfahren vermieden. Beispielsweise findet sich nach Durchführung des Verfahrens Vaseline im Adsorber, welche bei herkömmlichen Verfahren zu Ruß verbrennt.

Im Experiment wurden bislang Temperaturen bis knapp 300 °C eingestellt, ohne daß die erwartete Zersetzung auftrat. Der Einsatz solch hoher Temperaturen bewirkt eine nahezu vollständige Desorption des Lösungsmittels vom Adsorber 3 infolge der thermischen Anregung der Lösungsmittelmoleküle.

Im Anschluß an diese Hochtemperaturphase wird wieder abgekühlt und der desorbierte Stoff aus dem Adsorberraum 2 abgezogen. Das Abkühlen des desorbierten Stoffs mindestens bis auf eine Temperatur, bei welcher der Stoff kondensiert und einem wieder erhöhten Druck erfolgt in bekannter Weise und ist in der EP-A-0 281 941 beschrieben.

## Patentansprüche

1. Verfahren zur Rückgewinnung von in einem Adsorber (3) adsorbiertem Lösungsmittel, bestehend aus den Verfahrensschritten
(a) Erhitzen des Adsorbers (3) auf eine Temperatur, die unter der Zersetzungstemperatur des Lösungsmittels liegt,
(b) Abschließen des Adsorberraums (2) gegenüber der Umgebung,
(c) Anlegen eines hohen Unterdrucks an den Adsorberraum (2),
(d) Abziehen des desorbierten Lösungsmittels aus dem Adsorberraum (2) und
(e) Kondensieren des desorbierten Lösungsmittels durch Abkühlen bei geeignetem Druck,
**dadurch gekennzeichnet**, daß man während mindestens eines Teils der Zeit des Anliegens des hohen Unterdrucks an dem Adsorberraum (2) die Temperatur des Adsorbers (3) über die Zersetzungstemperatur des Lösungsmittels (bei normalem Luftdruck) erhöht.

2. Verfahren nach Anspruch 1, **dadurch gekennzeichnet**, daß das Abkühlen zuerst auf eine Temperatur erfolgt, die unter dem Gefrierpunkt des Lösungsmittels liegt und während der Temperaturerhöhung im Adsorberraum (2) die Abkühltemperatur unter dem Kondensationspunkt des Lösungsmittels liegt.

3. Verfahren nach Anspruch 1 oder 2, **dadurch gekennzeichnet**, daß das desorbierte Lösungsmittel in einen Kühlraum (10) transportiert wird, in dem es während seiner Abkühlung verweilt.

4. Verfahren nach einem der Ansprüche 1 bis 3, **dadurch gekennzeichnet**, daß der Raum, in dem sich der Adsorber (3) befindet, entlüftet wird, nachdem er abgeschlossen wurde und die Entlüftung von der Zuluftseite her erfolgt.

5. Verfahren nach Anspruch 3, **dadurch gekennzeichnet**, daß das desorbierte Lösungsmittel in dem Kühlraum (10) im Kreislauf über ein Kälteaggregat (11) geführt wird.

6. Verfahren nach einem der Ansprüche 1 bis 5, **dadurch gekennzeichnet**, daß die Abkühlung des desorbierten Lösungsmittels zweistufig erfolgt, wobei in der ersten Stufe unter die Kondensationstemperatur und in der zweiten Stufe unter den Erstarrungspunkt des desorbierten Lösungsmittels abgekühlt wird.

7. Verfahren nach einem der Ansprüche 1 bis 6, **dadurch gekennzeichnet**, daß der an den Absorberraum (2) angelegte Unterdruck den Wert von 10 mbar unterschreitet.

8. Verfahren nach Anspruch 7, **dadurch gekennzeichnet**, daß der an den Adsorberraum (2) angelegte Unterdruck den Wert von 1 mbar unterschreitet.

## Claims

1. A method of recovery of a solvent adsorbed in an adsorber (3), consisting of the following steps of the method:
(a) Heating the adsorber (3) up to a temperature which lies below the temperature of decomposition of the solvent;
(b) Closing off the adsorber chamber (2) from its surroundings;
(c) Applying a greatly reduced pressure to the adsorber chamber (2);
(d) Drawing off the desorbed solvent from the adsorber chamber (2); and
(e) Condensing the desorbed solvent by cooling down at suitable pressure;
characterized in that during at least part of the time of application of greatly reduced pressure to the adsorber chamber (2) the temperature of the adsorber (3) is raised (at normal air pressure) above the temperature of decomposition of the solvent.

2. A method as in Claim 1, characterized in that
the cooling down is at first effected to a temperature which lies below the freezing point of the solvent and during the raising of the temperature in the adsorber chamber (2) the cooling-down temperature lies below the condensation point of the solvent.

3. A method as in Claim 1 or 2, characterized in that
the desorbed solvent is conveyed into a cooling chamber (10) in which it dwells during its cooling down.

4. A method as in one of the Claims 1 to 3, characterized in that
the chamber in which the adsorber (3) lies is vented after it has been closed off and the venting is effected from the air inlet side.

5. A method as in Claim 3, characterized in that
the desorbed solvent is led into the cooling chamber (10) in the circuit via a refrigerator unit (11).

6. A method as in one of the Claims 1 to 5, characterized in that
the cooling down of the desorbed solvent is effected in two stages, so that In the first stage it is cooled down below the condensation temperature and in the second stage below the solidification point of the desorbed solvent.

7. A method as in one of the Claims 1 to 6, characterized in that
the reduced pressure applied to the adsorber chamber (2) lies below the value of 10 mbar.

8. A method as in Claim 7, characterized in that
the reduced pressure applied to the adsorber chamber (2) lies below the value of 1 mbar.

## Revendications

1. Procédé pour récupérer un solvant adsorbé dans un dispositif d'adsorption (3), constitué par les étapes de procédé suivantes :
(a) Chauffage du dispositif d'adsorption (3) jusqu'à une température située au-dessous de la température de décomposition du solvant,
(b) Fermeture de la chambre d'adsorption (2) par rapport à l'environnement,
(c) Application d'une forte dépression à la chambre d'adsorption (2),
(d) Extraction hors de la chambre d'adsorption (2) du solvant qui a été soumis à une désorption,
(e) Condensation par refroidissement sous une pression appropriée du solvant qui a été soumis à une désorption,
caractérisé par le fait que, du moins pendant une partie de la durée de l'application de la forte dépression à la chambre d'adsorption (2), on augmente la température du dispositif d'adsorption (3) jusqu'au-dessus de la température de décomposition du solvant (sous une pression atmosphérique normale).

2. Procédé selon la revendication 1, caractérisé par le fait que le refroidissement a lieu tout d'abord jusqu'à une température qui est située au-dessous du point de congélation du solvant, et que la température de refroidissement est située au-dessous du point de condensation du solvant pendant l'augmentation de la température dans la chambre d'adsorption (2)

3. Procédé selon la revendication 1 ou 2, caractérisé par le fait que le solvant qui a été soumis à une désorption est transporté dans une chambre de refroidissement (10) dans laquelle il séjourne pendant son refroidissement.

4. Procédé selon l'une des revendications 1 à 3, caractérisé par le fait que l'on évacue l'air de la chambre dans laquelle se trouve le dispositif d'adsorption (3) après qu'elle a été fermée, et que l'évacuation de l'air a lieu depuis le côté d'amenée de l'air.

5. Procédé selon la revendication 3, caractérisé par le fait que le solvant qui a été soumis à une désorption est amené dans la chambre de refroidissement (10) selon un circuit passant par un ensemble de refroidissement (11).

6. Procédé selon l'une des revendications 1 à 5, caractérisé par le fait que le refroidissement du solvant qui a été soumis à une désorption a lieu en deux phases, cependant que l'on refroidit jusqu'au-dessous de la température de condensation du solvant qui a été soumis à une désorption dans la première phase et jusqu'au-dessous de la température de solidification de celui-ci dans la seconde phase.

7. Procédé selon l'une des revendications 1 à 6, caractérisé par le fait que la dépression appliquée à la chambre d'adsorption (2) est inférieure à 10 mbars.

8. Procédé selon la revendication 7, caractérisé par le fait que la dépression appliquée à la chambre d'adsorption (2) est inférieure à 1 mbar.
